# EUROPEAN PATENT SPECIFICATION

(11) **EP 3 902 585 B1**
(45) Date of publication and mention of the grant of the patent: **19.02.2025**
(21) Application number: 19824355.2
(22) Date of filing: 20.12.2019
(51) Int. Cl.: A61M 5/19, A61M 5/20, A61M 5/24

(54) **DRUG RESERVOIR FOR SEPARATE STORAGE OF SUBSTANCES**
MEDIKAMENTENBEHÄLTER ZUR GETRENNTEN LAGERUNG VON SUBSTANZEN
RÉSERVOIR DE MÉDICAMENTS POUR STOCKAGE SÉPARÉ DE SUBSTANCES

(30) Priority: 28.12.2018 EP 18248210
(43) Date of publication of application: 03.11.2021
(73) Proprietor: Novo Nordisk A/S, 2880 Bagsværd (DK)
(72) Inventor: WINTHER, Knud Skifter, 2880 Bagsværd (DK)
(86) International application number: PCT/EP2019/086721
(87) International publication number: WO 2020/136124

(56) References cited:
- WO-A1-2012/090168
- WO-A1-2017/216651
- WO-A1-93/20867
- DE-A1- 102015 206 757
- JP-A- 2003 299 734
- JP-A- H0 775 673
- JP-B2- 3 246 998

## Description

### FIELD OF THE INVENTION

The present invention relates to reservoirs for medical use and particularly to reservoirs having more than one chamber.

### BACKGROUND OF THE INVENTION

Within some medical treatment areas a combination therapy involving co-administration of at least two drugs is advantageous because of synergistic or additive effects. For example, within diabetes care, in the management of type 2 diabetes mellitus, concomitant use of certain insulin and glp-1 products has been shown to reduce HbA_{1c} levels in subjects, thereby improving glycaemic control.

Many drugs must be administered parenterally to be effective in the body and some of these, e.g. insulin and glp-1, may require one or more doses to be delivered subcutaneously on a daily basis. Subcutaneous drug delivery is often associated with discomfort as many people dislike the thought of having an injection needle inserted through the skin. An undisclosed number of people even suffer from needle-phobia, and these people have a particularly strong desire to escape multiple daily injection therapy.

One attractive scenario, therefore, is to reduce the number of required skin penetrations by administering the drugs at the same time, or substantially the same time, through a single injection needle. In some cases, this is achievable by co-formulation of the active ingredients, where the co-formulated product is administered using a conventional injection device. In other cases, e.g. if the active ingredients are unsuitable for co-formulation, the individual substances are stored in separate chambers of a dual chamber, or multi-chamber, reservoir device from which they can be expressed, simultaneously or sequentially, through a single injection needle by use of dedicated expressing means.

US 4,394,863 (Survival Technology, Inc.) discloses an example of a dual chamber reservoir device in the form of an automatic injector with a cartridge having a fixedly mounted hypodermic needle. In a pre-use state of the device the cartridge holds a forward liquid medicament in a front chamber and a rearward liquid medicament in a rear chamber. The two liquids are separated by an intermediate piston, and the rear chamber is sealed proximally by a rearward piston. During use, in response to a release of a stressed spring, a plunger is urged forward, pushing the rearward piston and pressurising the rearward liquid medicament which transmits the movement of the rearward piston to the intermediate piston. Eventually, as the spring continues to provide a forward bias to the plunger, this leads to an expelling of the forward liquid medicament through the hypodermic needle, followed by an expelling of the rearward liquid medicament, via a distally arranged bypass section.

WO 2010/139793 (Novo Nordisk A/S) discloses an example of a dual chamber reservoir device in the form of a manually operated mixing device with a piston coupling arrangement allowing for an aspiration procedure to ensure proper insertion of an associated IV infusion needle. In a pre-use state of the device a dry drug, or a liquid, is held in a front chamber, and a liquid is held in a rear chamber. The two substances are separated by a front piston, and the rear chamber is sealed proximally by a rear piston through which a piston rod extends. During use the piston rod is manually advanced, slaving the rear piston and pressurising the rear chamber liquid which transmits the movement of the rear piston to the front piston. As the user continues to press the piston rod forward the front piston enters a bypass section and becomes immobilised because the pressure now forces the rear chamber liquid into the bypass, past the front piston and into the front chamber. In the front chamber the two substances mix as the rear chamber collapses. When the rear piston eventually reaches the front piston and the substances are thoroughly mixed the user can expel the mixed substance by continued advancement of the piston rod.

Documents JP H07 75673 A and WO 93/20867 A1 both disclose dual chamber reservoir devices.

A common drawback of such devices is the fact that during storage, over time, the piston material tends to adhere to the reservoir material, which means that a significant static friction must be overcome in order to initiate a drug mixing and/or expelling. Due to the incompressibility of the liquid in the rear chamber the two pistons will move in unison until the front piston reaches the bypass section. Resultantly, the force required to overcome this static friction is actually the sum of the forces required to break loose the individual pistons.

In case of a manually driven piston rod the sudden shift from static to kinetic friction as the pistons break loose is likely to cause a jerking forward motion of the pistons as the user tries to compensate for the sudden acceleration by significantly decreasing the force input. Apart from being an unpleasant user experience it may in fact lead to an overly fast transfer of the rear chamber liquid to the front chamber. If the front chamber carries a dry powder to be reconstituted the transfer process may even lead to undesired foaming.

In connection with spring driven injection devices like the automatic injector of US 4,394,863 the spring needs to be relatively powerful to ensure availability of a sufficient break-loose force. A downside of this is that once the friction becomes kinetic the power available for the actual drug expelling is very high and may lead to an unpleasantly high speed of delivery. Adding to that, a powerful spring requires stronger interfacing injection device parts to avoid creep or breakage during a potential medium- or long-term storage period in pre-loaded state, increasing both the cost and the weight of the injection device.

### SUMMARY OF THE INVENTION

It is an object of the invention to eliminate or reduce at least one drawback of the prior art, or to provide a useful alternative to prior art solutions.

In particular, it is an object of the invention to provide a drug reservoir for use in an injection device for delivery of more than one substance, where serially arranged pistons in the drug reservoir may be moved by application of a reduced force.

It is a further object of the invention to provide a drug delivery device for delivery of a plurality of substances through a single needle interface on the basis of a relatively small force input.

It is also an object of the invention to provide a cost-effective automatic injection device for delivery of a plurality of initially separated substances.

It is an even further object of the invention to provide a method for filling a drug reservoir having more than one chamber.

In the disclosure of the present invention, aspects and embodiments will be described which will address one or more of the above objects and/or which will address objects apparent from the following text.

In one aspect the invention provides a drug reservoir according to claim 1.

Hence, a drug reservoir is provided which comprises a reservoir body extending along a reference axis between a proximal end and an outlet end, a front piston arranged, in a pre-use position, within the reservoir body between the proximal end and the outlet end, a rear piston arranged within the reservoir body between the front piston and the proximal end, a distal chamber defined by the outlet end, a first portion of the reservoir body, and the front piston, a proximal chamber defined by the front piston, a second portion of the reservoir body, and the rear piston, and bypass means allowing fluid flow past the front piston in a particular advanced position of the front piston, i.e. distally of the pre-use position. The distal chamber holds first contents, e.g. comprising a distal liquid volume or a dry powder, and the proximal chamber holds second contents comprising a proximal liquid volume and a proximal gas volume. The proximal gas volume is a volume of non-liquid-bound gas, i.e. free gas which is not embedded on a molecular level in the liquid, lying within a volume range having a preset minimum value.

The non-liquid-bound gas is present as a gas volume between a surface portion of the proximal liquid volume and a surface portion of the proximal chamber, or as a gas bubble in the proximal liquid volume. Being non-liquid-bound the proximal gas volume provides resilience to the proximal chamber, in the sense that the second contents becomes compressible, as opposed to if the second contents consisted of liquid only. This has the effect that the two pistons will be broken loose sequentially instead of simultaneously. When a force of sufficient magnitude is applied to the rear piston the rear piston will be able to break loose from the inner wall of the reservoir body, whereby the force resisting movement of the rear piston will shift from a static friction force to a, lower, kinetic friction force, before the applied force is transferred to the front piston via the second contents. The break loose force needed to mobilise the two pistons is thus lower than if the second contents are incompressible and two static friction forces must be overcome at the same time.

As a consequence, when used in a drug delivery device the drug reservoir provides for a lower activation force during a drug expelling operation. For automatic injection devices, for example, this means that a less powerful spring may be employed to drive the drug expelling mechanism. A less powerful spring will in a pre-loaded state strain the interfacing device components less, reducing the risk of creep or breakage and/or allowing for use of less deformation resistant materials and/or or configurations, thereby reducing the cost of the drug delivery device.

The predetermined, or preset, minimum value of the volume range within which the proximal gas volume lies reflects the amount of gas needed to obtain the above described effect. This minimum value may depend on the transversal dimension of the reservoir body and the design of the rear piston. For drug reservoirs of the types and sizes commonly used in injection or infusion therapy such as that realised by subcutaneous self-administration of drugs, including reservoirs specified in *ISO 11040-4 (2015): Prefilled syringes - part 4: Glass barrels for injectables and ready-to-use prefillable syringes,* e.g. employing rubber pistons with an outer configuration as specified in *ISO 11040-5 (2012): Prefilled syringes - part 5: Plunger stoppers for injectables,* the inventors have established that a minimum gas volume of 15µl (e.g. for a standard 1 ml long PFS having an inner diameter of 6,35 mm) is required to provide sufficient resilience in the proximal chamber, allowing for a sequential release of the two pistons.

The volume range may further have a predetermined, or preset, maximum value, in which case the volume range is a predetermined closed volume range. The maximum value may reflect the maximum amount of gas guaranteed to not cause stability issues with the proximal liquid volume and/or certain practical considerations of the manufacturer, e.g. regarding the physical dimensions of the end product.

A maximum gas volume satisfying requirements to the physical size of the drug reservoir may, for example, be 200µl, 100µl, 75µl, or 50µl.

An optimum volume range may be established or approximated in view of the aforementioned requirements as well as the effect produced by a specific proximal gas volume. The inventors have determined that in some cases a predetermined volume range of [15µl; 200µl] is preferable, while in other cases e.g. a predetermined volume range of [20µl; 50µl] is preferable.

In some embodiments of the invention the first contents comprise a distal liquid volume and a distal (non-liquid-bound) gas volume, where the distal gas volume is smaller than the proximal gas volume.

The distal liquid volume may contain or comprise a first drug substance, and the proximal liquid volume may contain or comprise a second drug substance.

In some embodiments of the invention the proximal gas volume comprises air. This is particularly attractive in relation to the manufacturing of the drug reservoir, as the reservoir body may be filled in a conventional cleanroom environment.

In other embodiments of the invention the proximal gas volume comprises an inert gas to reduce the risk of undesired chemical reactions with the proximal liquid volume. In those embodiments the reservoir body may be filled in a cleanroom environment of the inert gas.

The drug reservoir may for example be a cartridge type reservoir, where a penetrable septum closes the outlet end, or a syringe type reservoir. In case of the latter the drug reservoir may further comprise a staked hollow needle, i.e. a hollow needle fixedly arranged at the outlet end and fluidly connected with the distal chamber. The hollow needle may be sealed off by a removable plug.

The bypass means may e.g. comprise a bypass channel as conventionally known from dual chamber medicament containers such as the one disclosed in US 4,394,863.

In another aspect of the invention a drug delivery device is provided comprising a drug reservoir as described in the above. The drug delivery device may further comprise a dose expelling structure for pressurising the proximal chamber. The dose expelling structure may comprise an actuatable piston rod adapted to transfer an expelling force to the rear piston.

The piston rod may be attached, or attachable, to the rear piston and adapted for direct manipulation by a user of the drug delivery device. Alternatively, the drug reservoir may be coupled with, e.g. embedded in, a housing of the drug delivery device and the piston rod may be operable via other components in the housing.

The dose expelling structure may be powered by a spring member operatively coupled with the piston rod and adapted to store energy releasable to urge the piston rod towards the outlet end. This will provide an automatic drug delivery device capable of executing a drug expelling with a minimum of user effort.

The drug delivery device may further comprise a retention structure which when enabled retains the spring member in a tensioned state, and a sleeve member extending axially along a portion of the housing and comprising a release structure, where the sleeve member is configured for proximal displacement relative to the housing and the retention structure from a first position in which the retention structure is enabled to a second position in which the retention structure is disabled by the release structure and stored energy consequently is released from the spring member.

In the first position the sleeve member may extend a distance beyond the outlet end such that a hollow needle arranged at the outlet end is covered by a distal end portion thereof. Thereby, the sleeve member may function as a combined needle shield and trigger for the tensioned spring.

Hence, by simply placing the sleeve on the skin and pressing the housing towards the skin the user will initiate an automatic injection because the release structure, during movement of the sleeve member to the second position, will disable the retention structure, thereby causing a release of stored energy from the spring, which energy is used to urge the piston rod distally relative to the housing, applying a force to the rear piston that causes a cascade of events including a compression of the second contents and a breaking loose of the rear piston from the inner wall of the reservoir body, a further pressurisation of the second contents and a breaking loose of the front piston from the inner wall of the reservoir body, an axial displacement of the proximal chamber until the front piston reaches the particular advanced position, during which a portion of the first contents may have been expelled through the outlet end, a collapse of the proximal chamber as the second contents are forced past the front piston via the bypass means, and finally an emptying, or substantial emptying, of the distal chamber.

Notably, the size and power of the spring required to accomplish this may be smaller than with prior art drug reservoirs due to the initial presence of the proximal gas volume, as described above.

In a further aspect of the invention a method of filling a drug reservoir comprising a generally cylindrical main body with a bypass section, a closed outlet end, and an open end is provided. The method comprises (i) arranging the drug reservoir at least substantially vertically with the open end facing upward, (ii) introducing a first liquid volume into the drug reservoir through the open end such that a first interior portion of the generally cylindrical main body, including the bypass section, is covered by liquid in a vertical position of the drug reservoir where the open end faces upward, (iii) in a first sub-atmospheric pressure environment inserting a first piston into the generally cylindrical main body to a first piston position at least substantially adjoining the free surface of the first liquid volume, thereby establishing a front chamber holding the first liquid volume, (iv) introducing a second liquid volume into the drug reservoir through the open end, and (v) in a second sub-atmospheric pressure environment of a surrounding gas inserting a second piston into the generally cylindrical main body to a second piston position, thereby establishing a rear chamber, where the second piston position is determined such that the rear chamber holds the second liquid volume and a rear chamber gas volume lying within a volume range having a predetermined minimum value.

The first sub-atmospheric pressure environment ensures that a negative pressure is established in the front chamber which will pull the first piston towards the free surface of the first liquid volume, minimising a present front chamber gas volume.

The second sub-atmospheric pressure environment may be established in a surrounding gas selected by the manufacturer in accordance with the constitution of the second liquid volume, e.g. air or an inert gas. The latter may be chosen to minimise the risk of undesired chemical reactions with the second liquid volume.

The second sub-atmospheric pressure environment ensures that a negative pressure is established in the rear chamber, which negative pressure may be controlled in order to place the second piston at the desired position within the generally cylindrical main body that, in view of the position of the first piston and the introduced second liquid volume, provides for a presence of gas in the predetermined minimum amount.

In some exemplary embodiments of the invention the predetermined minimum value is 15µl. In other exemplary embodiments of the invention the predetermined minimum value is 20µl.

In step (v) the second piston position may be determined such that the rear chamber gas volume lies within a predetermined closed volume range, i.e. such that the volume range further has a predetermined maximum value.

In some exemplary embodiments of the invention the predetermined maximum value is 200µl. In other exemplary embodiments of the invention the predetermined maximum value is 50µl.

The first piston and/or the second piston may be arranged in the desired piston position using an insertion tube having an external diameter which is smaller than an internal diameter of the generally cylindrical main body such that the insertion tube may be introduced through the open end and into the generally cylindrical main body without establishing physical contact thereto. The piston in question is then pre-arranged slidably within the insertion tube, and the insertion tube is inserted into the generally cylindrical main body to a position proximally of the desired piston position, whereupon the piston is slid out through a distal insertion tube opening and brought into sealing contact with an interior wall portion of the generally cylindrical main body.

Many drug reservoirs used in injection or infusion therapy have a siliconized interior surface to improve the friction interface to a piston. By using an insertion tube the piston may be inserted without sliding along the interior surface of the drug reservoir and resultantly scraping off the silicone. Scraped off silicone may over time interact with the liquid drug substance and cause precipitation, in which case the product is rendered useless.

For the avoidance of any doubt, in the present context the terms "distal" and "proximal" denote positions at, or directions along, a drug delivery device, or a needle unit, where "distal" refers to the drug outlet end and "proximal" refers to the end opposite the drug outlet end.

In the present specification, reference to a certain aspect or a certain embodiment (e.g. "an aspect", "a first aspect", "one embodiment", "an exemplary embodiment", or the like) signifies that a particular feature, structure, or characteristic described in connection with the respective aspect or embodiment is included in, or inherent of, at least that one aspect or embodiment of the invention, but not necessarily in/of all aspects or embodiments of the invention. It is emphasized, however, that any combination of the various features, structures and/or characteristics described in relation to the invention is encompassed by the invention unless expressly stated herein or clearly contradicted by context.

The use of any and all examples, or exemplary language (e.g., such as, etc.), in the text is intended to merely illuminate the invention and does not pose a limitation on the scope of the same, unless otherwise claimed. Further, no language or wording in the specification should be construed as indicating any non-claimed element as essential to the practice of the invention.

### BRIEF DESCRIPTION OF THE DRAWINGS

In the following the invention will be further described with references to the drawings, wherein
Fig. 1 is a longitudinal section view of a drug reservoir according to an exemplary embodiment of the invention in a pre-use state,
Figs. 2a-2c are graphs showing an initial force application to the rear reservoir piston in cases without a proximal gas volume, respectively with two different proximal gas volumes,
Fig. 3 is a principle sketch of the process for filling the drug reservoir with two liquid volumes,
Fig. 4 is a longitudinal section view of an exemplary drug delivery device employing the drug reservoir of Fig. 1,
Fig. 5 is a longitudinal section view of the drug delivery device in a ready to use state,
Figs. 6-10 are longitudinal section views of the drug delivery device in different in-use states, and
Fig. 11 is a longitudinal section view of the drug delivery device in a post use, emptied state.

In the figures like structures are mainly identified by like reference numerals.

### DESCRIPTION OF EXEMPLARY EMBODIMENTS

When/If relative expressions, such as "upper" and "lower", "left" and "right", "horizontal" and "vertical", "clockwise" and "counter-clockwise", etc., are used in the following, these refer to the appended figures and not necessarily to an actual situation of use. The shown figures are schematic representations for which reason the configuration of the different structures as well as their relative dimensions are intended to serve illustrative purposes only.

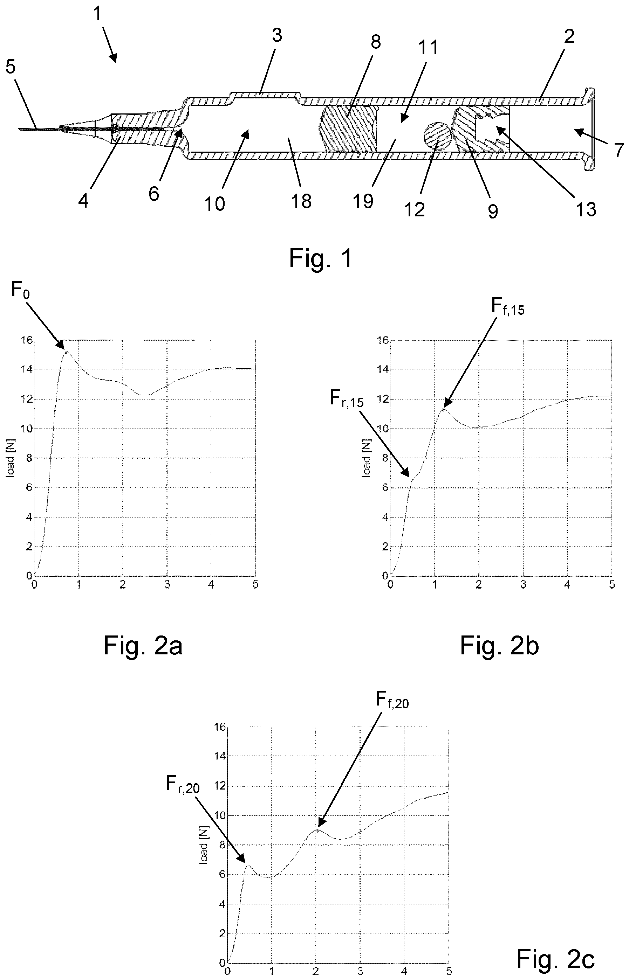

Fig. 1 is a longitudinal section view of a drug reservoir 1 according to an exemplary embodiment of the invention. The drug reservoir 1 is depicted in a pre-use state, i.e. in a state as supplied by the manufacturer (albeit without a rigid needle protector).

The drug reservoir 1 has a generally cylindrical reservoir body 2 with a bypass channel 3 and a narrowed distal end portion 4. An injection needle 5 is fixed to the distal end portion 4 and establishes fluid communication to a reservoir outlet 6. A front piston 8 is arranged in the reservoir body 2 between the reservoir outlet 6 and an open proximal end 7, and a front chamber 10 is thereby defined by the reservoir outlet 6, a front portion of the reservoir body 2 comprising the bypass channel 3, and the front piston 8. A rear piston 9 is arranged in the reservoir body 2 between the front piston 8 and the open proximal end 7, and a rear chamber 11 is thereby defined by the front piston 8, a middle portion of the reservoir body 2, and the rear piston 9. The rear piston 9 has a cavity 13 adapted to receive an end portion of a piston rod (not shown).

The front chamber 10 holds a first liquid substance 18, and the rear chamber 11 holds a second liquid substance 19 as well as a proximal gas volume 12, sketched in the form of a gas bubble in the liquid drug 19. The proximal gas volume 12 is deliberately introduced in the rear chamber 11 in order to reduce the force required to perform an expelling of the reservoir contents through the injection needle 5, as will be described in further detail below. In the present example the proximal gas volume is 15µl.

If a piston rod is inserted into the cavity 13 and a distally directed force is applied to the rear piston 9 the rear piston 9 will stay in its initial position until the applied force exceeds a certain threshold required to overcome the static friction in the contact interface between the sealing exterior surface of the rear piston 9 and the inner wall of the reservoir body 2.

Figs. 2a-2c indicate the initial force required to set the rear piston 9 into motion in three different cases, where the graph in Fig. 2a is the force profile for a drug reservoir without a proximal gas volume, the graph in Fig. 2b is the force profile for a drug reservoir with a proximal gas volume of 15µl, and the graph in Fig. 2c is the force profile for a drug reservoir with a proximal gas volume of 20µl.

In a dual chamber drug reservoir without a proximal gas volume in the rear chamber the liquid acts as a rigid connection between the front piston and the rear piston. The single force peak, F₀, in Fig. 2a reflects the fact that, in such a device, in order to set the rear piston into motion the front piston needs to be set into motion also, due to the incompressibility of the liquid. According to the present experiments a break loose force of approximately 15N is required to overcome the static friction in the system comprising both pistons.

In contrast thereto, as the graph in Fig. 2b shows, when a predetermined proximal gas volume of 15µl is present in the rear chamber 11 the gas will add some flexibility to the system which will result in the rear piston 9 breaking loose before the front piston 8. A smaller force, F_{r,15}, just short of 7N is required in this case to set the rear piston 9 into motion, as the proximal gas volume is compressed. The force rises subsequently, as the gas becomes fully compressed and the liquid/gas system consequently acts as a rigid connection between the two pistons, until the front piston 8 breaks loose at a next force peak, F_{f,15}, around 11N. A sudden drop in the force level following the breaking loose of the front piston 8 reflects the transition from static friction to kinetic friction between the pistons and the inner reservoir wall. As the liquid in the front chamber 10 is pressurised and forced out through the small lumen of the injection needle 5 at a continued motion of the front piston 8, the force again increases some, due to the flow resistance in the injection needle 5, but does not approach the level of F₀.

**In** **Fig. 2c** the difference is even more pronounced. Depicting results of experiments with a proximal gas volume of 20µl in the rear chamber 11, the graph reveals a comparable force, F_{r,20}, for breaking loose the rear piston 9 but a significantly smaller force, F_{r,20}, in the area of 9N, for subsequently breaking loose the front piston 8. All in all, as the graphs indicate, when a proximal gas volume of at least 15µl is present in the rear chamber 11 the required maximum force for initiating and carrying through a drug expelling action is reduced because the flexibility provided by said gas volume enables the rear piston 9 to break loose from the reservoir wall separately from the front piston 8.

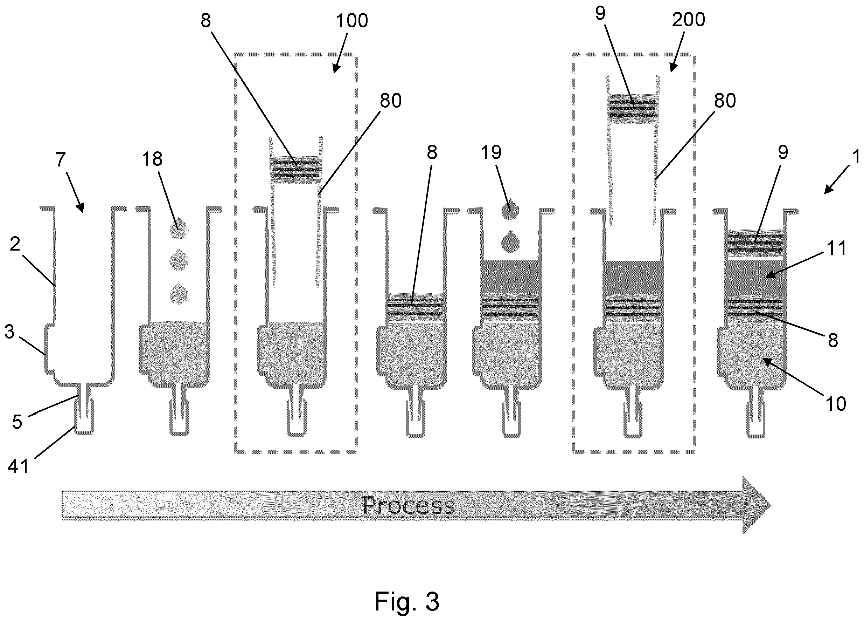

Fig. 3 is a principle sketch of the process for filling the drug reservoir 1 with two liquid volumes. From left to right the process steps include holding the drug reservoir 1 in an upright position with the injection needle 5 sealed up by a needle plug 41 and introducing a predetermined volume of the first liquid substance 18 into the reservoir body 2 through the proximal end 7.

Having filled a distal portion of the reservoir body 2 to a level where the bypass channel 3 is covered the drug reservoir 1 is placed in a first sub-atmospheric pressure environment 100. The front piston 8 is arranged in a radially compressed state in an insertion tube 80 having an inner diameter which is smaller than the inner diameter of the reservoir body 2, and the insertion tube 80 is introduced into the reservoir body 2 through the proximal end 7, notably without touching the inner wall of the reservoir body 2. The front piston 8 is then pushed through the insertion tube 80 and expands into contact with the inner wall of reservoir body 2 just above the free surface of the first liquid substance 18, thereby establishing the front chamber 10, and the drug reservoir 1 is subsequently re-exposed to normalised pressure conditions. The negative pressure in the front chamber 10 due to the front piston 8 being inserted in the first sub-atmospheric pressure environment 100 will cause the front piston 8 to move towards the first liquid substance 18, closing any gap to the free surface thereof.

A predetermined volume of the second liquid substance 19 is introduced into the reservoir body 2 though the proximal end 7 and fills a space above the front piston 8. The drug reservoir 1 is then placed in a second sub-atmospheric pressure environment 200 of a surrounding gas, and the insertion tube 80, now carrying the rear piston 9 in a radially compressed state, is introduced into the reservoir body 2 in a manner similar to the above described. This time the pressure is controlled such that when the rear piston 9 is deposited in the reservoir body 2, thereby establishing the rear chamber 11, and the drug reservoir 1 is subsequently re-exposed to normalised pressure conditions a volume of the surrounding gas remains in the rear chamber 11 as a free gas volume lying within a volume range having a predetermined minimum value.

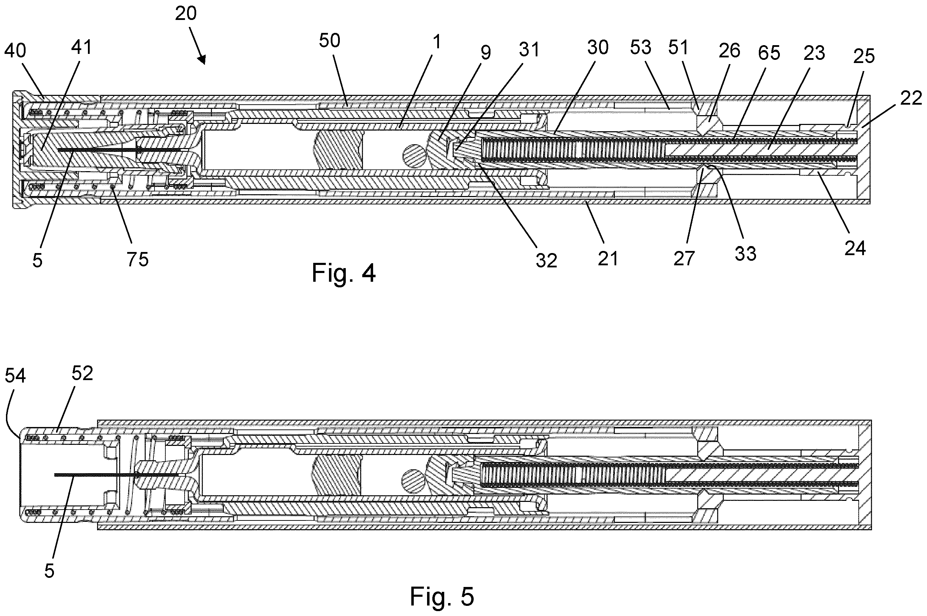

Fig. 4 is a longitudinal section view of the drug reservoir 1 forming part of an exemplary, dedicated auto-injector 20. The auto-injector 20 comprises a tubular housing 21 closed proximally by a transversal end wall 22 and accommodating a drug expelling mechanism including a piston rod 30 having a head portion 31 inserted into the cavity 13 and a shoulder portion 32 adapted to apply a distally directed force to the rear piston 9.

A couple of snap arms 24 extend distally from the transversal end wall 22 into the interior of the housing 21, ending in respective claws 26 with "v"-shaped interfacing portions 27 configured for engagement with corresponding depressions 33 in the piston rod 30. Each snap arm 24 has a proximal carving 25 which provides flexibility and allows for radial deflection of the claw 26.

A pre-tensioned compression spring 65 is arranged within the piston rod 30 and supported proximally by a central pin 23 which extends distally from the transversal end wall 22. The spring 65 is adapted to act between a distal end portion of the piston rod 30 and the transversal end wall 22.

The drug reservoir 1 is held within the housing 21 and is closed distally by a rigid needle protector 40 carrying the needle plug 41. An elongated sleeve 50 is arranged concentrically with, and between, the drug reservoir 1 and the housing 21. The sleeve 50 is axially displaceable relative to the housing 21, biased in the proximal direction by a sleeve spring 75, and comprises a radially enlarged proximal end portion 51 with a narrow adjoining section 53. In the shown pre-use state of the auto-injector 20 the sleeve 50 is in its maximum extended position relative to the housing 21, and the proximal end portion 51 is axially aligned with the claws 26, physically preventing the interfacing portions 27 from leaving the depressions 33. The auto-injector 20 is thus safely cocked, as the spring 65 is maintained in its pre-tensioned state because the piston rod 30 is unable to undergo axial motion relative to the housing 21.

Fig. 5 is a longitudinal section view of the auto-injector 20 in a ready-to-use state, after removal of the rigid needle protector 40 and the needle plug 41. The sleeve 50 is still in its maximum extended position relative to the housing 21, where a distal sleeve end portion 52 covers the injection needle 5 and thus protects the user from accidental needle stick injuries.

The distal sleeve end portion 52 has a sleeve rim 54 adapted to abut, and be pressed against, the user's skin at the desired injection site during drug expelling.

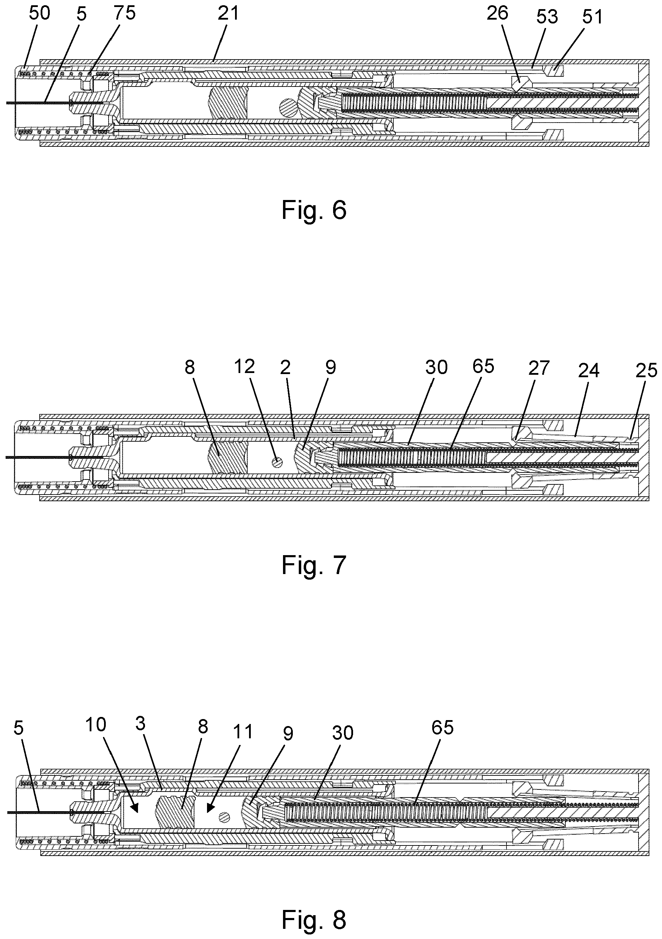

Figs. 6-11 illustrate in a step-wise manner the dose expelling sequence of the auto-injector 20. Firstly, the user places the sleeve rim 54 in contact with a desired skin location (not shown) and presses the housing 21 against the skin. This causes the distal sleeve end portion 52 to compress the sleeve spring 75, as the housing 21 and the sleeve 50 undergo relative axial motion from the mutual position shown in fig. 5 to that shown in Fig. 6. In essence the sleeve 50 is displaced proximally relative to the housing 21 and this causes the respective enlarged proximal end portions 51 to slide proximally along the claws 26. At some point, when the distal sleeve end portion 52 is pressed back sufficiently far that the tip of the injection needle 5 is exposed and has penetrated the skin surface, the sleeve 50 reaches a position relative to the snap arms 24 in which the enlarged proximal end portions 51 are no longer axially aligned with the claws 26. Instead, the claws 26 are axially aligned with the narrow adjoining section 53 and thereby no longer prevented from radial displacement.

The pre-tensioned spring 65 constantly provides a distally directed bias to the piston rod 30, so when the claws 26 are no longer radially fixated the axial force from the spring 65 and the respective configurations of the interfacing portions 27 and the depressions 33 will cause the snap arms 24 to deflect radially about the proximal carvings 25, leading to a disengagement of the claws 26 from the piston rod 30 and a resultant release of the spring 65. This is indicated in Fig. 7.

The initial result of the release of the spring 65 is also seen in Fig. 7. The presence of the proximal gas volume 12 enables a small compression of the rear chamber 11, so as the force from the expanding spring 65 pushes the piston rod 30 forward the rear piston 9 breaks loose from the inner wall of the reservoir body 2 while the front piston 8 remains stationary, the spring 65 at this point thus having to overcome only the static friction between the rear piston 9 and the reservoir body 2 (and not also the static friction between the front piston 8 and the reservoir body 2). In Fig. 7 the compression of the rear chamber 11 is illustrated by a reduced size of the proximal gas volume 12.

When the proximal gas volume 12 is fully compressed the contents of the rear chamber 11 will transfer the force from the spring 65 to the front piston 8 which will then break loose and move distally in the reservoir body 2 along with the rear piston 9, the second liquid substance 19 and the compressed proximal gas volume. The rear chamber 11 as such is thus displaced within the reservoir body 2, while a volume of the first liquid substance 18 is forced out through the injection needle 5, until the front piston 8 reaches the bypass channel 3, as shown in Fig. 8, at which point the second liquid substance 19 is forced into the bypass channel 3 and past the front piston 8 as the spring 65 keeps expanding.

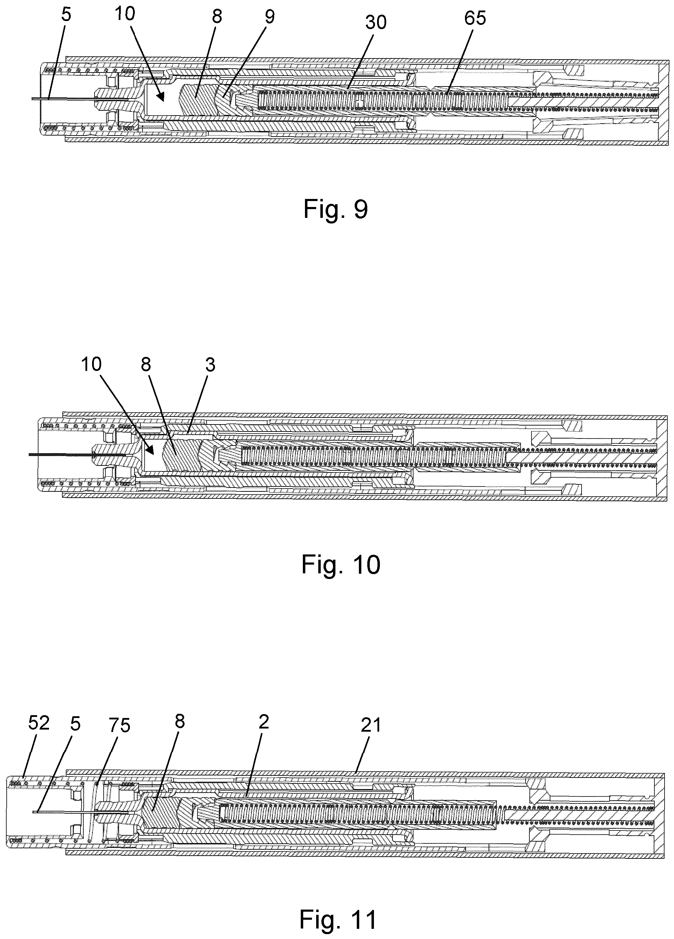

The rear chamber 11 eventually collapses as the rear piston 9 approaches the front piston 8 and the second liquid substance 19 is transferred to the front chamber 10 where it mixes with the remains of the first liquid substance 18. Fig. 9 depicts the state of the auto-injector 20 immediately after the collapse of the rear chamber 11.

The mixed first liquid substance 18 and second liquid substance 19 is now expelled from the front chamber 10 through the injection needle 5 as the rear piston 9, under the influence of the piston rod 30 and the spring 65, pushes the front piston 8 further distally in the reservoir body 2. In Fig. 10 the front piston 8 covers the distal end of the bypass channel 3 and thus seals off the front chamber 10 in the proximal direction.

The drug expelling continues until the front piston 8 reaches a constriction of the reservoir body 2 at the reservoir outlet 6, after which the injection needle 5 is pulled out of the skin by the user moving the housing 21 away from the injection site. As the pressure between the skin surface and the sleeve rim 74 is relieved the sleeve spring 75 expands and urges the sleeve 50 distally relative to the housing 21 until the distal sleeve end portion 52 again covers the injection needle 5. The auto-injector 20 is now in a post-use state, as shown in Fig. 11, and may be discarded safely with no risk of accidental needle stick injuries.

## Claims

1. A drug reservoir (1) comprising:
- a reservoir body (2) extending between an outlet end (4) and a proximal end (7),
- a front piston (8) arranged in a pre-use position within the reservoir body (2) between the outlet end (4) and the proximal end (7),
- a rear piston (9) arranged within the reservoir body (2) between the front piston (8) and the proximal end (7),
- a distal chamber (10) defined by the outlet end (4), a first portion of the reservoir body (2), and the front piston (8), the distal chamber (10) holding first contents (18),
- a proximal chamber (11) defined by the front piston (8), a second portion of the reservoir body (2), and the rear piston (9), the proximal chamber (11) holding second contents (12, 19) comprising a proximal liquid volume (19), and
- bypass means (3) allowing fluid flow past the front piston (8) in an advanced position of the front piston (8) in the reservoir body (2),
**characterised in that** the second contents (12, 19) further comprises a proximal gas volume (12) lying within a volume range having a predetermined minimum value.

2. A drug reservoir according to claim 1, wherein the predetermined minimum value is 15µl.

3. A drug reservoir according to claim 1, wherein the volume range is a predetermined closed volume range.

4. A drug reservoir according to claim 3, wherein the predetermined closed volume range is [15µl; 200µl].

5. A drug reservoir according to claim 3, wherein the predetermined closed volume range is [20µl; 50µl].

6. A drug reservoir according to any of the preceding claims, wherein the first contents (18) comprises a distal liquid volume and a distal gas volume, and wherein the distal gas volume is smaller than the proximal gas volume (12).

7. A drug reservoir according to any of the preceding claims, wherein the proximal gas volume (12) comprises air.

8. A drug reservoir according to any of claims 1 - 6, wherein the proximal gas volume (12) comprises an inert gas.

9. A drug reservoir according to any of the preceding claims, further comprising a hollow needle (5) fixedly arranged at the outlet end (4) and fluidly connected with the distal chamber (10).

10. A drug delivery device (20) comprising:
- a drug reservoir (1) according to any of the preceding claims, and
- a dose expelling structure for pressurising the proximal chamber (11), the dose expelling structure comprising an actuatable piston rod (30) adapted to transfer an expelling force to the rear piston (9).

11. A drug delivery device according to claim 10, further comprising a housing (21) extending along a reference axis, wherein the dose expelling structure is powered by a spring member (65) operatively coupled with the piston rod (30) and adapted to store energy releasable to urge the piston rod (30) towards the outlet end (4).

12. A drug delivery device according to claim 11, further comprising:
- a retention structure (24, 26, 27) which when enabled retains the spring member (65) in a tensioned state, and
- a sleeve member (50) extending axially along a portion of the housing (21) and comprising a release structure (51, 53),
wherein the sleeve member (50) is configured for proximal displacement relative to the housing (21) and the retention structure (24, 26, 27) from a first position in which the retention structure (24, 26, 27) is enabled to a second position in which the retention structure (24, 26, 27) is disabled by the release structure (51, 53) and stored energy consequently is released from the spring member (65).

13. A method of filling a drug reservoir (1) comprising a generally cylindrical main body (2) with a bypass section (3), a closed outlet end (4), and an open end (7), the method comprising:
(i) arranging the drug reservoir (1) at least substantially vertically with the open end (7) facing upward,
(ii) introducing a first liquid volume (18) into the drug reservoir (1) through the open end (7) such that a first interior portion of the generally cylindrical main body (2), including the bypass section (3), is covered by liquid in a vertical position of the drug reservoir (1) where the open end (7) faces upward,
(iii) in a first sub-atmospheric pressure environment (100) inserting a first piston (8) into the generally cylindrical main body (2) to a first piston position at least substantially adjoining the free surface of the first liquid volume (18), thereby establishing a front chamber (10) holding the first liquid volume (18),
(iv) introducing a second liquid volume (19) into the drug reservoir (1) through the open end (7), and
(v) in a second sub-atmospheric pressure environment (200) of a surrounding gas inserting a second piston (9) into the generally cylindrical main body (2) to a second piston position, thereby establishing a rear chamber (11), where the second piston position is determined such that the rear chamber (11) holds the second liquid volume (19) and a rear chamber gas volume (12) lying within a volume range having a predetermined minimum value.

## Patentansprüche

1. Arzneimittelbehälter (1), umfassend:
- einen Behälterkörper (2), der sich zwischen einem Auslassende (4) und einem proximalen Ende (7) erstreckt,
- einen vorderen Kolben (8), der in einer Vorgebrauchsposition innerhalb des Behälterkörpers (2) zwischen dem Auslassende (4) und dem proximalen Ende (7) angeordnet ist,
- einen hinteren Kolben (9), der innerhalb des Behälterkörpers (2) zwischen dem vorderen Kolben (8) und dem proximalen Ende (7) angeordnet ist,
- eine distale Kammer (10), die durch das Auslassende (4), einen ersten Abschnitt des Behälterkörpers (2) und den vorderen Kolben (8) definiert ist, wobei die distale Kammer (10) erste Inhalte (18) enthält,
- eine proximale Kammer (11), die durch den vorderen Kolben (8), einen zweiten Abschnitt des Behälterkörpers (2) und den hinteren Kolben (9) definiert ist, wobei die proximale Kammer (11) zweite Inhalte (12, 19) enthält, die ein proximales Flüssigkeitsvolumen (19) umfassen, und
- Bypassmittel (3), die in einer vorgeschobenen Position des vorderen Kolbens (8) in dem Behälterkörper (2) eine Fluidströmung an dem vorderen Kolben (8) vorbei ermöglichen,
**dadurch gekennzeichnet, dass** die zweiten Inhalte (12, 19) ferner ein proximales Gasvolumen (12) umfassen, das in einem Volumenbereich liegt, der einen vorbestimmten Minimalwert aufweist.

2. Arzneimittelbehälter nach Anspruch 1, wobei der vorbestimmte Minimalwert 15 µl beträgt.

3. Arzneimittelbehälter nach Anspruch 1, wobei der Volumenbereich ein vorbestimmter geschlossener Volumenbereich ist.

4. Arzneimittelbehälter nach Anspruch 3, wobei der vorbestimmte geschlossene Volumenbereich [15µl; 200µl] beträgt.

5. Arzneimittelbehälter nach Anspruch 3, wobei der vorbestimmte geschlossene Volumenbereich [20µl; 50µl] beträgt.

6. Arzneimittelbehälter nach einem der vorhergehenden Ansprüche, wobei die ersten Inhalte (18) ein distales Flüssigkeitsvolumen und ein distales Gasvolumen umfassen und wobei das distale Gasvolumen kleiner als das proximale Gasvolumen (12) ist.

7. Arzneimittelbehälter nach einem der vorhergehenden Ansprüche, wobei das proximale Gasvolumen (12) Luft umfasst.

8. Arzneimittelbehälter nach einem der Ansprüche 1-6, wobei das proximale Gasvolumen (12) ein inertes Gas umfasst.

9. Arzneimittelbehälter nach einem der vorhergehenden Ansprüche, ferner umfassend eine Hohlnadel (5), die fest an dem Auslassende (4) angeordnet ist und mit der distalen Kammer (10) in Fluidverbindung steht.

10. Arzneimittelabgabevorrichtung (20), umfassend:
- einen Arzneimittelbehälter (1) nach einem der vorhergehenden Ansprüche und
- eine Dosisausstoßstruktur zum Unterdrucksetzen der proximalen Kammer (11), wobei die Dosisausstoßstruktur eine betätigbare Kolbenstange (30) umfasst, die dazu ausgelegt ist, eine Ausstoßkraft auf den hinteren Kolben (9) zu übertragen.

11. Arzneimittelabgabevorrichtung nach Anspruch 10, ferner umfassend ein Gehäuse (21), das sich entlang einer Referenzachse erstreckt, wobei die Dosisausstoßstruktur durch ein Federelement (65) angetrieben wird, das betriebsmäßig mit der Kolbenstange (30) gekoppelt und dazu ausgelegt ist, Energie zu speichern, die freigesetzt werden kann, um die Kolbenstange (30) in Richtung des Auslassendes (4) zu treiben.

12. Arzneimittelabgabevorrichtung nach Anspruch 11, ferner umfassend:
- eine Haltestruktur (24, 26, 27), die, wenn sie aktiviert ist, das Federelement (65) in einem gespannten Zustand hält, und
- ein Hülsenelement (50), das sich axial entlang eines Abschnitts des Gehäuses (21) erstreckt und eine Freigabestruktur (51, 53) umfasst,
wobei das Hülsenelement (50) für eine proximale Verschiebung in Bezug auf das Gehäuse (21) und die Haltestruktur (24, 26, 27) konfiguriert ist, aus einer ersten Position, in der die Haltestruktur (24, 26, 27) aktiviert ist, in eine zweite Position, in der die Haltestruktur (24, 26, 27) durch die Freigabestruktur (51, 53) deaktiviert ist und demzufolge gespeicherte Energie von dem Federelement (65) freigegeben wird.

13. Verfahren zum Befüllen eines Arzneimittelbehälters (1), der einen im Allgemeinen zylindrischen Hauptkörper (2) mit einem Bypassabschnitt (3), ein geschlossenes Auslassende (4) und ein offenes Ende (7) umfasst, das Verfahren umfassend:
(i) Anordnen des Arzneimittelbehälters (1) zumindest im Wesentlichen vertikal, wobei das offene Ende (7) nach oben weist,
(ii) Einführen eines ersten Flüssigkeitsvolumens (18) in den Arzneimittelbehälter (1) durch das offene Ende (7), sodass ein erster Innenabschnitt des im Allgemeinen zylindrischen Hauptkörpers (2) einschließlich des Bypassabschnitts (3) in einer vertikalen Position des Arzneimittelbehälters (1), in der das offene Ende (7) nach oben weist, mit Flüssigkeit bedeckt ist,
(iii) in einer ersten Umgebung (100) mit weniger als Atmosphärendruck, Einführen eines ersten Kolbens (8) in den im Allgemeinen zylindrischen Hauptkörper (2) in eine erste Kolbenposition, die zumindest im Wesentlichen an die freie Oberfläche des ersten Flüssigkeitsvolumens (18) angrenzt, wodurch eine vordere Kammer (10) geschaffen wird, die das erste Flüssigkeitsvolumen (18) enthält,
(iv) Einführen eines zweiten Flüssigkeitsvolumens (19) in den Arzneimittelbehälter (1) durch das offene Ende (7) und,
(v) in einer zweiten Umgebung (200) mit weniger als Atmosphärendruck eines umgebenden Gases, Einführen eines zweiten Kolbens (9) in den im Allgemeinen zylindrischen Hauptkörper (2) in eine zweite Kolbenposition, wodurch eine hintere Kammer (11) geschaffen wird, wobei die zweite Kolbenposition so bestimmt wird, dass die hintere Kammer (11) das zweite Flüssigkeitsvolumen (19) und ein Gasvolumen (12) der hinteren Kammer enthält, das in einem Volumenbereich liegt, der einen vorbestimmten Minimalwert aufweist.

## Revendications

1. Réservoir de médicament (1) comprenant :
- un corps de réservoir (2) s'étendant entre une extrémité de sortie (4) et une extrémité proximale (7),
- un piston avant (8) agencé dans une position de pré-utilisation au sein du corps de réservoir (2) entre l'extrémité de sortie (4) et l'extrémité proximale (7),
- un piston arrière (9) agencé au sein du corps de réservoir (2) entre le piston avant (8) et l'extrémité proximale (7),
- une chambre distale (10) définie par l'extrémité de sortie (4), une première portion du corps de réservoir (2), et le piston avant (8), la chambre distale (10) maintenant un premier contenu (18),
- une chambre proximale (11) définie par le piston avant (8), une deuxième portion du corps de réservoir (2) et le piston arrière (9), la chambre proximale (11) maintenant un deuxième contenu (12, 19) comprenant un volume de liquide proximal (19), et
- des moyens de dérivation (3) permettant l'écoulement de fluide au-delà du piston avant (8) dans une position avancée du piston avant (8) dans le corps de réservoir (2),
**caractérisé en ce que** le deuxième contenu (12, 19) comprend en outre un volume de gaz proximal (12) situé au sein d'une plage de volume ayant une valeur minimale prédéterminée.

2. Réservoir de médicament selon la revendication 1, dans lequel la valeur minimale prédéterminée est de 15 µL.

3. Réservoir de médicament selon la revendication 1, dans lequel la plage de volume est une plage de volume fermée prédéterminée.

4. Réservoir de médicament selon la revendication 3, dans lequel la plage de volume fermée prédéterminée est [15 µL ; 200 µL].

5. Réservoir de médicament selon la revendication 3, dans lequel la plage de volume fermée prédéterminée est [20 µL ; 50 µL].

6. Réservoir de médicament selon l'une quelconque des revendications précédentes, dans lequel le premier contenu (18) comprend un volume de liquide distal et un volume de gaz distal, et dans lequel le volume de gaz distal est plus petit que le volume de gaz proximal (12).

7. Réservoir de médicament selon l'une quelconque des revendications précédentes, dans lequel le volume de gaz proximal (12) comprend de l'air.

8. Réservoir de médicament selon l'une quelconque des revendications 1 à 6, dans lequel le volume de gaz proximal (12) comprend un gaz inerte.

9. Réservoir de médicament selon l'une quelconque des revendications précédentes, comprenant en outre une aiguille creuse (5) agencée de manière fixe au niveau de l'extrémité de sortie (4) et raccordée fluidiquement à la chambre distale (10).

10. Dispositif d'administration de médicament (20) comprenant :
- un réservoir de médicament (1) selon l'une quelconque des revendications précédentes, et
- une structure d'expulsion de dose pour mettre sous pression la chambre proximale (11), la structure d'expulsion de dose comprenant une tige de piston actionnable (30) adaptée pour transférer une force d'expulsion au piston arrière (9).

11. Dispositif d'administration de médicament selon la revendication 10, comprenant en outre un logement (21) s'étendant le long d'un axe de référence, dans lequel la structure d'expulsion de dose est mise en marche par un organe de ressort (65) couplé de manière fonctionnelle à la tige de piston (30) et adaptée pour stocker de l'énergie libérable pour pousser la tige de piston (30) vers l'extrémité de sortie (4).

12. Système d'administration de médicament selon la revendication 11, comprenant en outre :
- une structure de retenue (24, 26, 27) qui, lorsqu'elle est activée, retient l'organe de ressort (65) dans un état tendu, et
- un organe de manchon (50) s'étendant axialement le long d'une portion du logement (21) et comprenant une structure de libération (51, 53),
dans lequel l'organe de manchon (50) est configuré pour un déplacement proximal par rapport au logement (21) et à la structure de retenue (24, 26, 27) à partir d'une première position dans laquelle la structure de retenue (24, 26, 27) est activée à une deuxième position dans laquelle la structure de retenue (24, 26, 27) est désactivée par la structure de libération (51, 53) et l'énergie stockée est, par conséquent, libérée de l'organe de ressort (65).

13. Procédé de remplissage d'un réservoir de médicament (1) comprenant un corps principal (2) généralement cylindrique avec une section de dérivation (3), une extrémité de sortie fermée (4) et une extrémité ouverte (7), le procédé comprenant :
(i) l'agencement du réservoir de médicament (1) au moins sensiblement verticalement avec l'extrémité ouverte (7) tournée vers le haut,
(ii) l'introduction d'un premier volume de liquide (18) dans le réservoir de médicament (1) à travers l'extrémité ouverte (7) de telle sorte qu'une première portion intérieure du corps principal (2) généralement cylindrique, comprenant la section de dérivation (3), est recouverte de liquide dans une position verticale du réservoir de médicament (1) où l'extrémité ouverte (7) est tournée vers le haut,
(iii) dans un premier environnement de pression subatmosphérique (100), l'insertion d'un premier piston (8) dans le corps principal (2) généralement cylindrique vers une première position de piston au moins sensiblement attenante à la surface libre du premier volume de liquide (18), établissant ainsi une chambre avant (10) maintenant le premier volume de liquide (18),
(iv) l'introduction d'un deuxième volume de liquide (19) jusque dans le réservoir de médicament (1) à travers l'extrémité ouverte (7), et
(v) dans un deuxième environnement de pression subatmosphérique (200) d'un gaz environnant, l'insertion d'un deuxième piston (9) dans le corps principal (2) généralement cylindrique jusqu'à une deuxième position de piston, établissant ainsi une chambre arrière (11), où la deuxième position de piston est déterminée de telle sorte que la chambre arrière (11) maintient le deuxième volume de liquide (19) et un volume de gaz de chambre arrière (12) se trouvant au sein d'une plage de volume ayant une valeur minimale prédéterminée.
